# EUROPEAN PATENT SPECIFICATION

(11) **EP 1 468 690 B1**
(45) Date of publication and mention of the grant of the patent: **08.10.2008**
(21) Application number: 03075894.0
(22) Date of filing: 27.03.2003
(51) Int. Cl.: A61K 31/565, A61K 38/11, A61P 15/08

(54) **Use of estrogens for the treatment of infertility in male mammals**
Verwendung von Estrogenen zur Behandlung von Männerunfruchtbarkeit
Utilization d' oestrogenes dans le traitement de la sterilite masculine

(43) Date of publication of application: 20.10.2004
(73) Proprietor: Pantarhei Bioscience B.V., 3701 CH Zeist (NL)
(72) Inventor: Maas, Jacob Jan, 1066 WP Amsterdam (NL); Coelingh Bennink, Herman Jan Tijmen, 3985 MK WERKHOVEN (NL); Nieschlag, Eberhard, 48167 Münster (DE)
(74) Representative: Jorritsma, Ruurd

(56) References cited:
- WO-A-00/18424
- WO-A-00/63228
- WO-A-01/77139
- WO-A-98/43660
- WO-A-98/43661
- ASTERIA C: "Importance of estrogens in human males' fertility and bone pathophysiology." EUROPEAN JOURNAL OF ENDOCRINOLOGY / EUROPEAN FEDERATION OF ENDOCRINE SOCIETIES. ENGLAND AUG 1997, vol. 137, no. 2, August 1997 (1997-08), pages 120-121, XP009015407 ISSN: 0804-4643

## Description

### TECHNICAL FIELD OF THE INVENTION

The present invention relates to treatment or prevention of infertility of a male mammal, wherein the treatment comprises administering to said male a fertility agent in an amount effective to increase the quality of the male's semen, especially in terms of sperm count, ejaculate volume, sperm motility and/or sperm morphology.

### BACKGROUND OF THE INVENTION

Infertility affects millions of couples. The female partner is apparently normal in some 60 percent of those couples affected. Couples with no evident factors causing infertility, having intercourse at the time of ovulation every month, will only become pregnant once in every 4.5 months i.e. a pregnancy rate of 22% per month. This is why infertility is not considered until a couple has been trying to become pregnant for at least a year. Around 20% of all couples will have failed to achieve pregnancy within that period and will face investigation and treatment for infertility. Infertility has a wide range of causes which need to be eliminated by tests and investigations in both partners. No cause will, however, be found in around 20% of infertile couples and their infertility will be classified as idiopathic or unexplained infertility.

In case of male factor deficiency the male may be diagnosed as being infertile or subfertile due to poor semen characteristics. Generally, a total sperm count of less than approximately 40 million/ejaculate, which is defined as the product of ejaculate volume and sperm density, or alternatively a sperm concentration of less than 20 million/ml, with adequate motility and morphology, is considered to be a low sperm count, and males exhibiting such characteristics are considered to be oligozoospermic. Oligozoospermia is considered to be severe when sperm count is lower than 5 million/ml. Astenozoospermia refers to spermatozoa of very weak mobility, or none at al, teratozoospermia refers to a spermocytogram with less than 40% normal sperm, azoospermia refers to no spermatozoa in the ejaculate at all and finally parvisemia refers to an ejaculate volume of less than 2 ml.

Primary infertility is usually defined as the inability to conceive after one year of unprotected intercourse, whereas secondary infertility as the inability to conceive again after one successful conception.

"Germinal epithelium failure" refers to a disorder of mammals that exhibit oligozoospermia as described above and yet have intact Leydig cell steroidogenic capacity and pituitary function. Such males have normal testosterone levels but low or non-existent sperm counts. In the former case, the disorder is generally referred to as "partial germinal epithelium failure" while in the latter case, the disorder is generally referred to as "complete germinal epithelium failure". There are various causes for this including; genetic, tract infections and varicoceles. In addition, the natural ageing process may have some effect upon the production of sperm, decreasing in older males.

The term "unexplained" or "idiopathic" infertility is applied to every couple whose failure to conceive within a period of at least one year, is clinically inexplicable and where fertility testing of both partners has revealed no identifiable cause. When a couple is found to be "normal" in a standard fertility evaluation (female - ovulatory and normal post-coital tests, timed endometrial biopsy, hysterosalpingogram, laparoscopy; male - "normal" sperm analysis, with sperm concentration greater than 20 million/ml on at least two occasions, total sperm numbers of 40 million or more, sperm motility greater than 50%, and normal morphology in more than 30% of the spermatozoa), and the couple has had a history of involuntary infertility for at least 1 year, a human couple is diagnosed unsatisfactorily as "unexplainably" or "idiopathic" infertile. Such couples often undergo numerous invasive, protracted and expensive assisted reproductive technology attempts in their pursuit of pregnancy.

Current treatments for male infertility include induction of rebound from testosterone or anabolic androgen-induced azoospermia; administration of exogenous gonadotrophins or gonadotrophin releasing hormone; use of clomiphene citrate or tamoxifen to stimulate endogenous gonadotrophin secretion; administration of low doses of mesterolone, an oral synthetic androgen; and use of an aromatase inhibitor such as testolactone.

Testosterone rebound therapy involves large doses of testosterone that suppress the activity of the patient's pituitary gland. This, in tum, reduces the intratesticular level of testosterone to systemic levels. Next, androgen therapy is discontinued in the hope that the system will rebound and improved spermatogenesis will result. This therapy is not recommended since a large number of treated patients continue to exhibit azoospermia after treatment.

Human Chorionic Gonadotrophin (hCG) is administered empirically to patients with defects in sperm count or motility to correct a presumed intratesticular deficiency of testosterone. Some patients actually experienced an increase of sperm count. Urinary Human Menopausal Gonadotrophin (hMG) has approximately equal LH and FSH activity and has produced increased sperm counts in about 50% of cases. These favourable results, however, could not be confirmed in a placebo-controlled, prospective, double blind and randomised study (Knuth et al. J. Clin. Endocrinol Metab 65: 1081-7, 1987). FSH and hCG or hCG and hMG combination therapy does not appear to improve these results. Gonadotrophin Releasing Hormone (GnRH) is expensive and disappointing results have been obtained.

Antiestrogen drugs (such as clomlphene citrate and tamoxifen) are used to prevent sex hormones from exerting a negative feed back on FSH and LH secretion by the pituitary gland. This increases release of LH and FSH, which in turn stimulates testosterone production. Increased testosterone level improves spermatogenesis, thus improving sperm density and motility. However a randomised, double-blind, multicenter study of 190 couples by the World Health Organization (WHO) showed no effect of clomiphene citrate. Tamoxifen was claimed to improve sperm concentration but no change in motility was usually detected. As for clomiphene, recent studies did not confirm its efficacy.

Mesterolone is a synthetic androgen widely used to treat idiopathic male infertility. A study sponsored by WHO failed to show any efficacy of this drug.

Testolactone, an aromatase inhibitor, prevents the conversion of testosterone to estradiol. It has been tested in patients with idiopathic oligozoospermia but contrasting results have raised many doubts on its efficacy.

WO 00/18424 teaches that oxytocin can be used (i) to stimulate fetal growth, (ii) to enhance ovarian maturation function, (iii) to stimulate recovery of peripheral and central neuropathies, (iv) to stimulate human dermal dendritic cells, (v) to stimulate dermal keratinocytes, (vi) to stimulate the cortical bone thickness, (vii) to reduce apoptosis, (viii) to protect from ester-and oxazolone-induced inflammation, (ix) to modulate aged skin barrier formation, and (x) to reduce cell energy consumption. This international patent application further contains the following statement (page 3, lines 20-23): *It has now turned out that oxytocin* ...*enhances ovarian maturation and function (Example* 2*), i*.*e*. *an effect against sterility which is helpful in fertilisation in vitro and in vivo and to increase the sperm and egg production,* ...

WO 00/63228 describes ent-steroids that exhibit selective estrogenic activity. On page 24, third paragraph of this international patent application it is stated: *In addition, the compounds can be used for the treatment of male fertility disorders and prostatic diseases.*

WO 01/77139 describes 8β-hydrocarbyl-substituted estratrienes that exhibit selective estrogenic activity. On page 24, second paragraph of this international patent application it is stated: *In addition, the compounds can be used for the treatment of male fertility disorders and prostatic diseases.*

It is an object of the present invention to provide a fundamentally new, safe and efficacious treatment for increasing the fertility of male mammals, in particular of male mammals that suffer from oligozoospermia or azoospermia and of male mammals exhibiting normal sperm quality but suffering from "unexplained" or "idiopathic" infertility.

### SUMMARY OF THE INVENTION

The inventors have unexpectedly found that the fertility of male mammals can be improved significantly by administering to said male mammal a combination of estrogen and oxytocin agonist in an amount effective to increase the reproductive quality of the male's semen.

Although the inventors do not wish to be bound by theory, it is believed that estrogen and oxytocin receptors in epididymis and vas deferens fulfil a critical role in the post-gonadal modification of spermatozoa and the peristalsis of the reproductive tract (propulsion of sperm). It is hypothesised that administration of estrogen may be employed to increase expression of oxytocin receptors in the epididymis and/or vas deferens and to enhance the responsiveness of these organs to oxytocin. Thus, reduced fertility as a result of deficient post-gonadal modification (epididymis) and/or impaired peristalsis of the vas deferens can be remedied by administration of an adequate amount of estrogen. In addition, estrogen administration can reinforce the biological effect of oxytocine on spermatogenesis and testicular steroidogenesis. Finally, exogenous supply of estrogen may restore reduced estrogen plasma levels which are believed to be associated with deficient spermatogenesis and limited sperm transport within the epididymal tract.

Thus, the present treatment may be used advantageously to improve fertility of males whose fertility has been reduced by one or more of the aforementioned factors (especially spermatogenesis, post-gonadal modification and/or sperm transport). The improved fertility resulting from the present treatment is usually evident in parameters that are indicative of semen quality such as total sperm count, sperm concentration, ejaculate volume, sperm motility and/or sperm morphology.

The present treatment is particularly effective as it employs administering a combination of estrogen and oxytocin agonist. Co-administration of an oxytocin agonist ensures that the increased oxytocin responsiveness is actually utilised to enhance spermatogenesis, post-gonadal modification and/or sperm transport.

### DETAILED DESCRIPTION OF THE INVENTION

Accordingly, the present invention relates to the treatment or prevention of primary or secondary infertility of a male mammal, said treatment comprising administering to said male mammal a combination of estrogen and oxytocin agonist in an amount effective to increase the reproductive quality of the male's semen. Generally accepted parameters that are indicative of the reproductive quality of semen include total sperm count, sperm concentration, ejaculate volume, sperm motility and sperm morphology. Preferably, the present treatment is effective in significantly increasing at least one of these parampters, e.g. by at least 10%. More preferably the present treatment is effective in increasing at least one of said parameters by at least 30%, most preferably by at least 50%.

The treatment of the present invention may be used to improve the fertility of all kinds of mammals, including humans, farm animals and pets. Preferably, the present treatment is used to improve the fertility of humans or cattle, The present treatment is particularly suitable for treating human males, especially human males who are between 18 and 50 years old.

The present treatment requires the administration of a relatively small amount of estrogen. Typically, estrogen is administered in an amount equivalent to a daily oral dosage of between 0.01 mg and 5 mg 17β-estradiol. Preferably, estrogen is administered in an amount equivalent to a daily oral dosage of at least 0.03 mg 17β-estradiol, more preferably of at least 0.05 mg 17β-estradiol. The administered amount of estrogen preferably does not exceed an amount equivalent to a daily oral dosage of 1 mg 17β-estradiol, more preferably it does not exceed the equivalent of a daily oral dosage of 0.5 mg 17β-estradiol.

The present treatment may suitably employ any estrogen that is capable of triggering an *in vivo* estrogenic response. Examples of suitable estrogens include ethinyl estradiol, mestranol, quinestranol, estradiol, estrone, estran, estriol, estetrol, conjugated equine estrogens, precursors capable of liberating such an estrogen when used in the present treatment and mixtures thereof. Preferably, the estrogen used in the present treatment is selected from the group consisting of ethinyl estradiol, estradiol, estetrol, precursors capable of liberating such an estrogen when used in the present method and mixtures thereof.

The estrogen may suitably be administered enterally (e.g orally or rectally) or parenterally. More preferably, the estrogen is administered orally, intranasally, pulmonary, bucally, transdermally, subcutaneously or intramuscularly. Even more preferably, the estrogen is administered orally or transdermally. Most preferably the estrogen is administered transdermally.

Preferably, the present treatment is used for treating normogonadotrophic oligospermic males and/or for treating males who suffer from secondary idiopathic Infertility. Most preferably, the method is used for treating normogonadotrophic oligospermic males.

The present treamtent is particularly effective in increasing sperm count and/or ejaculate volume in the male mammal. More particularly, the present treatment is effective in increasing the total sperm number, which can be calculated by multiplying sperm density with ejaculate volume. In a very preferred embodiment the present treatment is used to increase sperm number by at least 30%, preferably by at least 50%.

The present treatment is especially suitable for treating male mammals that, according to commonly accepted definitions, are deemed to be infertile. Typically, such males, prior to administration, exhibit a total sperm count of less than 40 million/ejaculate.

In an especially preferred embodiment of the present invention the treatment comprises administering a synergistic combination of estrogen and oxytocin agonist in an amount effective to increase the quality of the sernen in terms of sperm count, ejaculate volume, sperm motility or sperm morphology. The combination of estrogen and oxytocin agonist is advantageously administered in a therapeutically effective amount to stimulate the emptying of the epidydimus via the ductus deferens. Improved emptying of the epidydimus is evidenced by increased sperm density and/or ejaculate volume.

The oxytocin agonist used in the present treatment may be any substance capable of binding to the oxytocin receptor and triggering a signal transduction event at said receptor. The oxytocin agonist is preferably selected from the group consisting of oxytocin, precursors capable of liberating oxytocin when used in the present treatment and combinations thereof. Most preferably the oxytocin agonist is oxytocin.

The oxytocin agonist may be administered enterally or parenterally. Preferably, the oxytocin agonist is administered orally, intranasally, pulmonary, bucally, transdermally, subcutaneously or intramuscularly. More preferably, the oxytocin agonist is administered intranasally, subcutaneously or intramuscularly. In a particularly preferred embodiment, estrogen is administered orally, intranasally, subcutaneously or intramuscularly. Estrogen and oxytocin agonist are advantageously administered in the same manner, especially intranasally, subcutaneously or intramuscularly. Most preferably both estrogen and oxytocin agonist are administered intramuscularly or subcutaneously.

Typically, in the present treatment the oxytocin agonist is administered in an amount of at least 2 µg, preferably of at least 10 µg, more preferably of at least 20 µg. Usually the administered amount does not exceed 500 µg, preferably it does not exceed 200 µg. Expressed differently, the oxytocin agonist is usually administered in an amount equivalent to a daily oral dosage of at least 2 µg oxytocin, preferably of at least 10 µg oxytocin and most preferably at least 20 µg oxytodn. The administered amount normally does not exceed the equivalent of a daily oral dosage of 80 µg oxytocin, preferably it does not exceed the equivalent of a daily oral dosage of 60 µg oxytocin. The strength of the preparations of synthetic oxytocin *(Syntocinon ®)* is often expressed in International Units (IU), each IU being the equivalent of approximately 2 µg of pure hormone.

In one embodiment of the invention, the present treatment employs administration of estrogen and oxytocin agonist at regular intervals. In this embodiment, depending on the mode of administration, estrogen and oxytocin agonist may be administered at intervals in the range of few hours to several weeks. Oral and intranasal delivery typically employ at least once daily administration, whereas transdermal patches may be applied e.g. once daily up to once weekly. Preferably, in a protocol that involves regular administration, estrogen and oxytocin agonist are administered at least once daily, most preferably once daily. Such a protocol of administration is advantageously continued for a period of at least 1 week, more preferably for a period of at least 2 weeks en most preferably for a period of at least 2 months.

In an alternative embodiment, the present treatment employs administration of estrogen and oxytocin antagonist within a short time period prior to coitus. Preferably, said time period does not exceed 4 hours, more preferably it does not exceed 2 hours and most preferably, administration occurs within 1 hour before coitus.

The invention is further illustrated by means of the following examples.

### EXAMPLES

### Example 1

A double-blind, randomised, cross-over study is conducted to investigate whether estrogen or oxytocin alone, or in combination will improve total sperm count and sperm volume in infertile, normogonadotropic, oligozoospermic men. Subjects are selected on the basis of the following criteria
• At least 18 years and not older than 40 years of age at the time of screening
• Involuntary infertility of at least 1 year
• Oligozoospermia (< 20 million spermatozoa/ml)
• Normal FSH(1-7 U/I)
• Testicular size >16 ml (Prader)
• Body Mass Index ≥18 and ≤29 kg/m²
• Good physical and mental health

The exclusion criteria are:
• Contraindications for steroids:
   - A history of, or existing thromboembolic, cardiovascular or cerebrovascular disorder
   - The presence of more than one risk factor for vascular disease (e.g. dyslipoproteinemia; diabetes mellitus; diabetes insipidus; hyperhomocysteinemia; systemic lupus erythematosus; chronic inflammatory bowel disease; antiphospholipid antibodies; smoking; venous thromboembolism in sibling or parent below the age of 50, or arterial disease in sibling or parent below the age of 30-35; within 2 weeks after full remobilisation following surgery)
   - Hypertension, i.e. systolic blood pressure >140 mm Hg and/or diastolic blood pressure >90 mm Hg
   - Disturbance of liver function
   - Porphyria
• Use of one or more of the following medications:
   - Sex steroids other than the medication of study
   - Medication for blocking endogenous androgens production
   - Use within 2 months before start study medication of
      hydantoins, barbiturates, primidone, carbamazepine, oxcarbazepine, topiramate, troglitazone, felbamate, rifampicin, rifabutin, griseofulvin, prostaglandines, sympathicometica, St. John's wort (Hypericum perforatum) or cytotoxic medication.
• Known hypersensitivity for one of the components of the study medication
• Administration of investigational drugs within 3 months before start study medication
• Known potential reasons for male infertility, such as a clinical varicocele or orchititis, maldescended testes, testicular tumours, diseases of the hypothalamus /pituitary gland and vasectomy
• Congenital and acquired illnesses associated with fertility and reproduction
• Previous or manifest malignant diseases
• A history of (within 12 months) alcohol or drug abuse.

This study is designed as a double-blind, randomised, two-period, cross-over study in which 30 Infertile, normogonadotropic, oligozoospermic, otherwise healthy, male subjects will be randomised into three groups.

### Group 1 (n = 10):

The subjects are treated alternatingly with a single dose of either 20 IU oxytocin (OT) *(Syntocinon®)* or placebo. Five subjects are randomised to receive an injection with 20 IU OT at the end of every two weeks of the first period of 6 weeks and a placebo injection at the end of every two weeks during the second period of 6 weeks, each injection immediately being followed by manual masturbation to produce a semen sample. The remaining 5 subjects will be randomised to first receive a placebo injection at the end of every two weeks of the first period of 6 weeks and an OT injection at the end of every two weeks during the second period of 6 weeks, each injection immediately being followed by manual masturbation to produce a semen sample.

### Group 2 (n=10)

The subjects are treated alternatingly with oral doses of 17β-estradiol (E2) or placebo. Five subjects are randomised to receive daily oral doses of 0.25 mg E2 for 6 weeks and a placebo during the second period of 6 weeks. After each period of 2 weeks a semen sample is obtained by manual masturbation. The remaining 5 subjects are randomised to first receive a placebo during 6 weeks and a daily oral dose of 0.25 mg E2 during the following 6 weeks. Also during the second period a semen sample is obtained by manual masturbation after every 2 weeks of treatment.

### Group 3 (n = 10):

All subjects are treated continuously for 12 weeks with 12.5 µg E2 per day, by means of a transdermal *Estradiol patch®.* Five subjects are randomised to receive concomitantly an injection with 20 IU OT at the end of every two weeks of the first period of 6 weeks and a placebo injection at the end of every two weeks during the second period of 6 weeks, each injection immediately being followed by manual masturbation to produce a semen sample. The remaining 5 subjects are randomised to first receive a placebo injection at the end of every two weeks of the first period of 6 weeks and an OT injection at the end of every two weeks during the second period of 6 weeks, each injection immediately being followed by manual masturbation to produce a semen sample.

The E2 transdermal patches used are obtained by cutting 50 µg/24 hrs *Estradzol patch®* (PCT Pharmachemie) into 4 patches of each 12.5 µg/24 hrs. The patches are worn in a continuous fashion, starting from the time of randomisation until the end of the study. The E2 patches are changed every three to four days according to the instruction for use in the package insert.

The results of the study show that administration of E2 and the administration of a combination of oxytocin and E2 induces favourable changes in sperm parameters, such as sperm density and ejaculate volume. Surprisingly, the concomitant administration of oxytocin and a low dose of E2 is found to improve the total sperm count in a synergistic way, i.e. the observed change is significantly more dramatic than could be expected on the basis of the effects of each individual component.

Additional evidence on the beneficial effect of the combined administration of oxytocin and E2 is seen as well. Administration of oxytocin in combination with E2 is found to have a favourable effect on motility. The improvement in sperm motility observed as a result of the combined administration of oxytocin in combination with E2 clearly exceeds the effects seen for either oxytocin or E2 alone. From these results it can be concluded that E2, and in particular the combination of oxytocin and E2, can be employed advantageously in the treatment of infertile, normogonadotropic, oligozoospermic men.

### Example 2

Example 1 is repeated using (10 µg) 20 IU oxytocin intranasally administered instead of 20 IU intramuscularly administered oxytocin. Similar results are obtained.

### Example 3

Example 1 is repeated using 10 µg EE orally per day instead of E2. Again similar results are obtained as in Example 1.

## Claims

1. Use of an estrogen in the manufacture of a pharmaceutical composition for use in a method of treating or preventing primary or secondary infertility of a male mammal, wherein the method comprises administering to said male mammal a combination of estrogen and oxytocin agonist in an amount effective to increase the reproductive quality of the male's semen.

2. Use according to claim 1, wherein the method is effective in increasing the reproductive quality of the male's semen in terms of total sperm count, sperm concentration, ejaculate volume, sperm motility and/or sperm morphology.

3. Use according to claim 1 or 2, wherein estrogen is administered in an amount equivalent to a daily oral dosage of between 0.01 mg and 5 mg 17β-estradiol.

4. Use according to any one of claims 1-3, wherein the estrogen employed in the combination of estrogen and oxytocin agonist is selected from the group consisting of ethinyl estradiol, mestranol, quinestranol, estradiol, estrone, estran, estriol, estetrol, conjugated equine estrogens and mixtures thereof.

5. Use according to any one of claims 1-4, wherein the estrogen is administered orally, intranasally, pulmonary, bucally, transdermally, subcutaneously or intramuscularly.

6. Use according to any one of claims 1-5 in a method of treating or preventing primary infertility, idiopathic infertility or secondary infertility in a male mammal.

7. Use according to any one of claims 1-6, wherein the method is effective in increasing sperm count and/or ejaculate volume in the male mammal.

8. Use according to any one of claims 1-7, wherein the male mammal, prior to administration, exhibits a sperm concentration of less than 20 million/ml.

9. Use according to any one of claims 1-8, wherein the method comprises administering a combination of estrogen and oxytocin agonist in an amount effective to increase the quality of the semen in terms of sperm count, semen volume, sperm motility or sperm morphology.

10. Use according to any one of claims 9, wherein the combination of estrogen and oxytocin agonist is administered in a therapeutically effective amount to stimulate the emptying of the epidydimus via the ductus deferens.

11. Use according to claim 9 or 10, wherein the oxytocin agonist is oxytocinand combinations thereof.

12. Use according to any one of claims 9-11, wherein the oxytocin agonist is administered orally, intranasally, pulmonary, bucally, transdermally, subcutaneously or intramuscularly.

13. Use according to any one of claims 9-12, wherein the oxytocin agonist is administered in an amount of at least 2 µg, preferably of at least 10 µg.

14. Use according to any one of claims 9-13, wherein the oxytocin agonist is administered in an amount equivalent to a daily intra muscular dosage of at least 2 µg oxytocin.

15. Use according to any one of claims 1-14, wherein estrogen is administered at least once daily.

16. Use according to any one of claims 1-14, wherein estrogen is administered at least 4 hours prior to coitus.

17. Use according to claim 16, wherein a combination of estrogen and oxytocin agonist is administered at least 4 hours prior to coitus.

## Patentansprüche

1. Verwendung eines Estrogens in der Herstellung einer pharmazeutischen Zusammensetzung zur Verwendung in einem Verfahren zum Behandeln oder Verhindern von primärer oder sekundärer Unfruchtbarkeit eines männlichen Säugers, wobei das Verfahren das Verabreichen einer Kombination von Estrogen und von Oxytocin-Agonist in einer Menge, welche eine Steigerung der reproduktiven Qualität des Samens des männlichen Wesens bewirkt, an besagten männlichen Säuger u m-fasst.

2. Verwendung gemäß Anspruch 1, wobei das Verfahren eine Steigerung der reproduktiven Qualität des Samens des männlichen Wesens im Hinblick auf Gesamtspermienzahl, Spermienkonzentration, Ejakulatvolumen, Spermienmotilität und/oder Spermienmorphologie bewirkt.

3. Verwendung gemäß Anspruch 1 oder 2, wobei Estrogen in einer Menge, die einer oralen Tagesdosierung von zwischen 0,01 mg und 5 mg 17β-Estradiol entspricht, verabreicht wird.

4. Verwendung gemäß einem der Ansprüche 1-3, wobei das Estrogen, das in der Kombination von Estrogen und von Oxytocin-Agonist eingesetzt wird, ausgewählt ist aus der Gruppe bestehend aus Ethinylestradiol, Mestranol, Quinestranol, Estradiol, Estron, Estran, Estriol, Estetrol, konjugierten, equinen Estrogenen und Gemischen davon.

5. Verwendung gemäß einem der Ansprüche 1-4, wobei das Estrogen oral, intranasal, pulmonal, buccal, transdermal, subcutan oder intramuskulär verabreicht wird.

6. Verwendung gemäß einem der Ansprüche 1-5 in einem Verfahren zum Behandeln oder Verhindern von primärer Unfruchtbarkeit, idiopathischer Unfruchtbarkeit oder sekundärer Unfruchtbarkeit in einem männlichen Säuger.

7. Verwendung gemäß einem der Ansprüche 1-6, wobei das Verfahren eine Steigerung der Spermienzahl und/oder des Ejakulatvolumens in dem männlichen Säuger bewirkt.

8. Verwendung gemäß einem der Ansprüche 1-7, wobei der männliche Säuger vor der Verabreichung eine Spermienkonzentration von weniger als 20 Millionen/ml aufweist.

9. Verwendung gemäß einem der Ansprüche 1-8, wobei das Verfahren das Verabreichen einer Kombination von Estrogen und Oxytocin-Agonist in einer Menge, die eine Steigerung der Qualität des Samens im Hinblick auf Spermienzahl, Samenvolumen, Spermienmotilität oder Spermienmorphologie bewirkt, umfasst.

10. Verwendung gemäß einem der Ansprüche 1-9, wobei die Kombination von Estrogen und Oxytocin-Agonist in einer therapeutisch wirksamen Menge verabreicht wird, um das Entleeren des Nebenhodens über den Ductus deferens zu stimulieren.

11. Verwendung gemäß Anspruch 9 oder 10, wobei es sich bei dem Oxytocin-Agonisten um Oxytocin oder Kombinationen davon handelt.

12. Verwendung gemäß einem der Ansprüche 9-11, wobei der Oxytocin-Agonist oral, intranasal, pulmonal, buccal, transdermal, subcutan oder intramuskulär verabreicht wird.

13. Verwendung gemäß einem der Ansprüche 9-12, wobei der Oxytocin-Agonist in einer Menge von mindestens 2 µg, vorzugsweise von mindestens 10 µg verabreicht wird.

14. Verwendung gemäß einem der Ansprüche 9-13, wobei der Oxytocin-Agonist in einer Menge, die einer täglichen intramuskulären Dosierung von mindestens 2 µg Oxytocin entspricht, verabreicht wird.

15. Verwendung gemäß einem der Ansprüche 1-14, wobei Estrogen mindestens einmal täglich verabreicht wird.

16. Verwendung gemäß einem der Ansprüche 1-14, wobei Estrogen mindestens 4 Stunden vor dem Koitus verabreicht wird.

17. Verwendung gemäß Anspruch 16, wobei eine Kombination von Estrogen und von Oxytocin-Agonist mindestens 4 Stunden vor dem Koitus verabreicht wird.

## Revendications

1. Utilisation d'un oestrogène dans la fabrication d'une composition pharmaceutique destinée à une utilisation dans une méthode de traitement ou de prévention d'une infertilité primaire ou secondaire d'un mammifère mâle, dans laquelle la méthode comprend l'administration audit mammifère mâle d'une combinaison d'oestrogène et d'agoniste de l'ocytocine en une quantité efficace pour augmenter la qualité reproductive du sperme du mâle.

2. Utilisation selon la revendication 1, dans laquelle la méthode est efficace pour augmenter la qualité reproductive du sperme du mâle en termes de numération totale des spermatozoïdes, concentration de spermatozoïdes, volume d'éjaculat, motilité du sperme et/ou morphologie du sperme.

3. Utilisation selon la revendication 1 ou 2, dans laquelle l'oestrogène est administré en une quantité équivalente à une dose orale quotidienne de 0,01 mg à 5 mg de 17β-oestradiol.

4. Utilisation selon l'une quelconque des revendications 1 à 3, dans laquelle l'oestrogène employé dans la combinaison d'oestrogène et d'agoniste de l'ocytocine est sélectionné dans le groupe constitué par l'éthinyl oestradiol, le mestranol, le quinestranol, l'oestradiol, l'oestrone, l'oestrane, l'oestriol, l'estertrol, les oestrogènes équins conjugués et des mélanges de ceux-ci.

5. Utilisation selon l'une quelconque des revendications 1 à 4, dans laquelle l'oestrogène est administré par voie orale, intranasale, pulmonaire, buccale, transdermique, sous-cutanée ou intramusculaire.

6. Utilisation selon l'une quelconque des revendications 1 à 5 dans une méthode de traitement ou de prévention de l'infertilité primaire, l'infertilité idiopathique ou l'infertilité secondaire chez un mammifère mâle.

7. Utilisation selon l'une quelconque des revendications 1 à 6, dans laquelle la méthode est efficace pour augmenter la numération des spermatozoïdes et/ou le volume d'éjaculat chez le mammifère mâle.

8. Utilisation selon l'une quelconque des revendications 1 à 7, dans laquelle le mammifère mâle, avant administration, présente une concentration de spermatozoïdes de moins de 20 millions/ml.

9. Utilisation selon l'une quelconque des revendications 1 à 8, dans laquelle la méthode comprend l'administration d'une combinaison d'oestrogène et d'agoniste de l'ocytocine en une quantité efficace pour augmenter la qualité du sperme en termes de numération des spermatozoïdes, volume de sperme, motilité du sperme ou morphologie du sperme.

10. Utilisation selon l'une quelconque des revendications 1 à 9, dans laquelle la combinaison d'oestrogène et d'agoniste de l'ocytocine est administrée en une quantité thérapeutiquement efficace pour stimuler l'évacuation de l'épididyme par le canal déférent.

11. Utilisation selon la revendication 9 ou 10, dans laquelle l'agoniste de l'ocytocine est l'ocytocine et des mélanges de celle-ci.

12. Utilisation selon l'une quelconque des revendications 9 à 11, dans laquelle l'agoniste de l'ocytocine est administré par voie orale, intranasale, pulmonaire, buccale, transdermique, sous-cutanée ou intramusculaire.

13. Utilisation selon l'une quelconque des revendications 9 à 12, dans laquelle l'agoniste de l'ocytocine est administré en une quantité d'au moins 2 µg, de préférence d'au moins 10 µg.

14. Utilisation selon l'une quelconque des revendications 9 à 13, dans laquelle l'agoniste de l'ocytocine est administré en une quantité équivalente à une dose intramusculaire quotidienne d'au moins 2 µg d'ocytocine.

15. Utilisation selon l'une quelconque des revendications 1 à 14, dans laquelle l'oestrogène est administré au moins une fois par jour.

16. Utilisation selon l'une quelconque des revendications 1 à 14, dans laquelle l'oestrogène est administré au moins 4 heures avant le coït.

17. Utilisation selon la revendication 16, dans laquelle une combinaison d'oestrogène et d'agoniste de l'ocytocine est administrée au moins 4 heures avant le coït.
